(19) Europäisches Patentamt
European Patent Office
Office européen des brevets

(11) Veröffentlichungsnummer: **0 095 047**
**B1**

# (12) EUROPÄISCHE PATENTSCHRIFT

(45) Veröffentlichungstag der Patentschrift: **27.11.85**

(51) Int. Cl.⁴: **C 07 C 69/747**, C 07 C 67/10, C 07 C 61/35, C 07 C 49/227, C 07 C 45/61

(21) Anmeldenummer: **83104022.5**

(22) Anmeldetag: **25.04.83**

(54) **Verfahren zur Herstellung von 3-vinylsubstituierten 2,2-Dimethylcyclopropan-1-carbonsäuren bzw. ihren Estern und neue Zwischenprodukte dafür.**

(30) Priorität: **05.05.82 DE 3216790**
**26.08.82 DE 3231814**

(43) Veröffentlichungstag der Anmeldung: **30.11.83 Patentblatt 83/48**

(45) Bekanntmachung des Hinweises auf die Patenterteilung: **27.11.85 Patentblatt 85/48**

(84) Benannte Vertragsstaaten: **BE CH DE FR GB IT LI NL**

(56) Entgegenhaltungen:
**DE - A - 2 605 398**
**DE - A - 2 621 835**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**
Erfinder: **Arlt, Dieter, Prof.Dr., Rybnikerstrasse 2, D-5000 Köln 80 (DE)**
Erfinder: **Jautelat, Manfred, Dr., Müllersbaum 28, D-5093 Burscheid (DE)**

**EP 0 095 047 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 3-vinylsubstituierten 2,2-Dimethylcyclopropan-1-carbonsäuren und -estern sowie neue Zwischenprodukte dafür und deren Herstellung.

Es ist bereits bekannt geworden, dass Ester von 3-Styryl-2,2-dimethyl-cyclopropancarbonsäuren insektizide Eigenschaften besitzen (z. B. Deutsche Offenlegungsschriften Nrn. 2706184, 2738150). Die Verbindungen werden hergestellt, indem man die entsprechenden Ylide in einer Wittig-Reaktion mit Caronaldehyd umsetzt (Deutsche Offenlegungsschriften Nrn. 2738150, 2837101). Diese Synthesen sind teuer, da Caronaldehyd nur schwer zugänglich ist. Auch auf anderen Wegen sind die Säuren nur über vielstufige Synthesewege zugänglich.

Es ist ausserdem bekannt geworden, dass Ester von 3-dihalogenvinylsubstituierten Cyclopropancarbonsäuren insektizide Eigenschaften besitzen (DE-OS Nr. 2326077).

Die Säuren werden z. B. hergestellt, indem man 1,1-Dihalogen-4-methyl-pentadien mit Diazoessigester umsetzt [Farkas *et al.*, „Col. Czech Comm. Chem.", 24, 2230 (1959)].

Sie können auch erhalten werden, indem Prenol und Orthoessigsäureester zu Dimethylpentensäureester umgesetzt, Tetrahalogenmethan addiert wird und mit Alkoholaten die entstandenen Verbindungen dehydrohalogeniert werden (DE-OS Nr. 2539895).

Diese Verfahren erfordern entweder hohe Sicherheitsvorkehrungen oder sie sind technisch aufwendig, da zahlreiche Stufen durchlaufen werden müssen. Ausserdem gehen diese Verfahren von verhältnismässig teuren Ausgangsprodukten aus.

Es wurde nun gefunden, dass man Verbindungen der Formel I

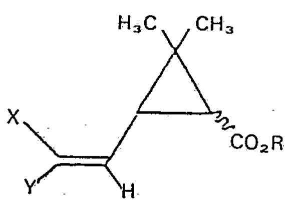
(I)

in welcher

Y für Halogen, Alkyl, Cycloalkyl, die ggf. durch Halogen oder $C_{1-4}$-Alkoxy substituiert sind, Alkenyl, das ggf. durch Halogen substituiert ist, für substituiertes oder unsubstituiertes Aryl oder Heteroaryl sowie für Alkoxycarbonyl steht,

X für Wasserstoff, Halogen oder ggf. durch Halogen substituiertes Alkyl steht,

wobei X und Y gemeinsam mit dem angrenzenden C-Atom für einen gesättigten cycloaliphatischen Ring mit bis zu 6 C-Atomen bilden können, und

R für Wasserstoff oder $C_1$-$C_4$-Alkyl steht,

erhält, indem man Aldehyde der Formel II

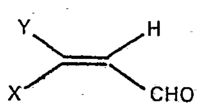
(II)

in welcher

Y und X die oben angegebene Bedeutung besitzen,

mit Methyl-butan-3-on (III)

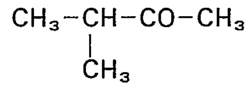
(III)

in Anwesenheit von Halogenwasserstoffsäuren umsetzt, die dabei erhaltenen 4,4-Dimethyl-3-halogen-1-hexen-5-one der Formel IV

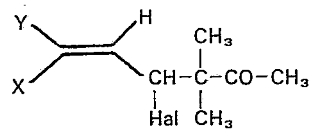
(IV)

in welcher

Y und X die oben angegebene Bedeutung besitzen und

Hal für Chlor oder Brom steht,

halogeniert und die dabei erhaltenen Verbindungen der Formel V

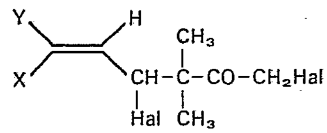
(V)

in welcher

Y, X und Hal die oben angegebene Bedeutung besitzen, wobei die beiden Hal unabhängig voneinander sind,

mit Basen der Formel VI

$$R{-}OM \qquad (VI)$$

in welcher

R die oben angegebene Bedeutung besitzt, und

M für ein Äquivalent eines Alkali- oder Erdalkalimetallions steht,

umsetzt.

Es wurden ferner die neuen 4,4-Dimethyl-3-halogen-1-hexen-5-one der Formel IV gefunden

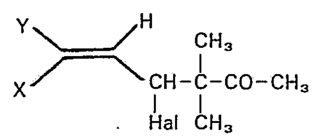
(IV)

in welcher

Y für Halogen, Alkyl, Cycloalkyl, die ggf. durch Halogen oder $C_1$-$C_4$-Alkoxy substituiert sind, Alkenyl, das ggf. durch Halogen substituiert ist, für substituiertes oder unsubstituiertes Aryl oder Heteroaryl sowie für Alkoxycarbonyl steht,

X für Wasserstoff, Halogen oder ggf. durch Halogen substituiertes Alkyl steht,

wobei X und Y gemeinsam mit dem angrenzenden C-Atom für einen gesättigten cycloaliphatischen Ring mit bis zu 6 C-Atomen stehen können, und

Hal für Chlor oder Brom steht.

Es wurde gefunden, dass man die 4,4-Dimethyl-3-halogen-1-hexen-5-one der Formel IV

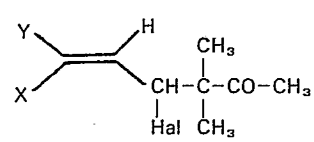
(IV)

in welcher

Y, X und Hal die unter 2. angegebene Bedeutung haben,

erhält, indem man Aldehyde der Formel II

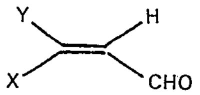

(II)

in welcher

Y und X die oben angegebene Bedeutung haben,

mit 2-Methyl-butan-3-on der Formel III

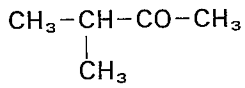

(III)

in Gegenwart von halogenwasserstoffsäuren umsetzt.

Es wurden ferner die neuen Verbindungen der Formel V gefunden

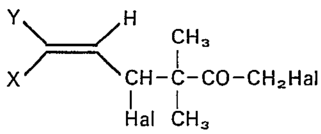

(V)

in welcher

Y für Halogen, Alkyl, Cycloalkyl, die ggf. durch Halogen oder $C_{1-4}$-Alkoxy substituiert sind, Alkenyl, das ggf. durch Halogen substituiert ist, für substituiertes oder unsubstituiertes Aryl oder Heteroaryl sowie für Alkoxycarbonyl steht,

X für Wasserstoff, Halogen oder ggf. durch Halogen substituiertes Alkyl steht,

wobei X und Y gemeinsam mit dem angrenzenden C-Atom für einen gesättigten aliphatischen Ring mit bis zu 6 C-Atomen stehen können,

und die beiden Hal unabhängig voneinander für Chlor oder Brom stehen.

Es wurde ferner gefunden, dass man Verbindungen der Formel V

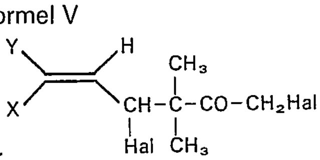

(V)

in welcher

Y, X und Hal die unter 4 (oben) angegebene Bedeutung haben,

erhält, indem man 4,4-Dimethyl-3-halogen-1-hexen-5-one der Formel IV

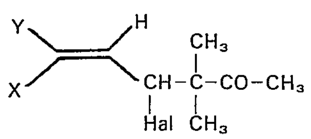

(IV)

in welcher

Y, X und Hal die oben angegebene Bedeutung haben, halogeniert.

Es wurde ferner gefunden, dass man Verbindungen der Formel I

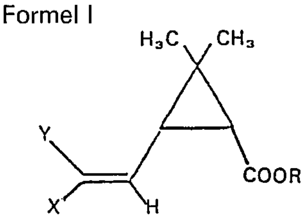

(I)

in welcher

Y, X und R die oben angegebene Bedeutung haben, erhält, indem man Verbindungen der Formel V

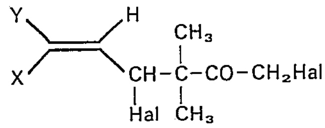

(V)

in welcher

Y, X und Hal die oben angegebene Bedeutung haben, mit Basen der Formel VI

R–O–M (VI)

in welcher

R und M die oben angegebene Bedeutung haben, umsetzt.

4,4-Dimethyl-3-halogen-1-hexen-5-one der Formel IV (oben) erhält man, wie bereits erwähnt, indem man Aldehyde der Formel II

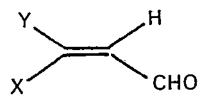

(II)

mit 2-Methyl-butan-3-on (III) in Gegenwart von Halogenwasserstoffen umsetzt.

Der Verlauf der Reaktion ist überraschend. Nach dem Stand der Technik konnte erwartet werden, dass die Aldehyde an der Methylgruppe des 2-Methyl-butan-3-ons angreifen.

So findet sich beispielsweise in „Organic Reactions", Bd. 16, S. 31 unten/32 oben die Voraussage, dass zu erwarten sei, dass säurekatalysierte Kondensationen von Aldehyden mit Ketonen an der Methylgruppe stattfinden. Es ist weiterhin bekannt, dass es entscheidend von der Struktur abhängt, ob Aldehyde an der $CH_3$- oder der CH-Gruppe reagieren. So reagieren Benzaldehyd und 4-Chlor-benzaldehyd mit Methylalkylketonen nur in Spuren an der CH-Gruppe und überwiegend an der $CH_3$-Gruppe. 4-Methoxybenzaldehyd dagegen reagiert mit Methylalkylketonen zu 70% an der CH-Gruppe [„Archiv für Pharmazie", *308*, 422 (1975)].

Verwendet man beispielsweise 3-Chlor-3-phenyl-propenal als Ausgangsstoff, so kann der Reaktionsablauf durch folgendes Formelschema wiedergegeben werden:

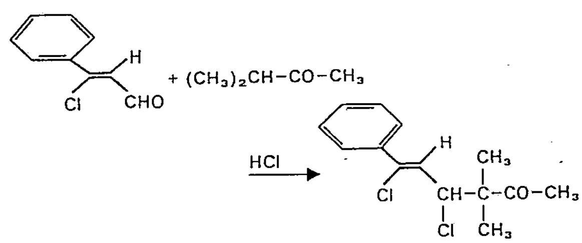


Die bei diesen Verfahren verwendeten Ausgangsstoffe sind durch die allgemeine Formel II definiert. Darin steht Y bevorzugt für: Halogen, insbesondere Fluor, Chlor, Brom, $C_{1-4}$-Alkyl, Cyclopropyl, die ggf. durch Halogen, insbesondere Fluor oder Chlor, substituiert sind, Dichlorvinyl, für ggf. durch Halogen, Cyan, $C_{1-4}$-Alkyl, $C_{1-4}$-Halogenalkyl, $C_{1-4}$-Alkoxy, $C_{1-4}$-Halogenalkoxy, $C_{1-4}$-Alkylthio, $C_{1-4}$-Halogenalkylthio sub-

stituiertes Phenyl, ggf. halogensubstituiertes Thiopen, sowie für $C_{1-4}$-Alkoxycarbonyl.

X steht bevorzugt für Wasserstoff oder Halogen, insbesondere Fluor, Chlor, Brom, sowie ggf. durch Fluor oder Chlor substituiertes $C_{1-4}$-Alkyl.

Besonders bevorzugt steht Y für ggf. durch Fluor, Chlor, Brom, Cyano, Methyl, Ethyl, Methoxy, Trifluormethyl, Trifluormethoxy, Trifluormethylmercapto substituiertes Phenyl sowie für Fluor, Chlor, Brom, Methyl, Trifluormethyl.

Ganz besonders bevorzugt steht Y für Chlor oder für chlorsubstituiertes Phenyl.

X steht bevorzugt für Fluor, Chlor, Brom oder Methyl.

Ganz besonders bevorzugt steht X für Chlor.

Die als Ausgangsstoffe zu verwendenden Aldehyde der Formel II sind bekannt oder können nach bekannten Methoden hergestellt werden (z. B. „Zeitschrift für Chemie", 1976, *16*, S. 337; „Houben Weyl" Bd. 7, I S. 119; EP-Pat Nr. 31041). Die ausserdem als Ausgangsprodukt einzusetzende Verbindung 2-Methyl-butan-1-on ist bekannt.

Die Reaktion wird in Gegenwart mindestens äquimolarer Mengen Chlorwasserstoff oder Bromwasserstoff durchgeführt.

Es kann mit oder ohne Verdünnungsmittel gearbeitet werden. Als Verdünnungsmittel kommen alle gegen Chlor- oder Bromwasserstoff inerten Lösungsmittel in Frage, wie beispielsweise Kohlenwasserstoffe, wie Cyclohexan, Petrolether, Benzol, Toluol oder Chlorkohlenwasserstoffe, wie Methylenchlorid, Chloroform, Tetrachlorkohlenstoff oder Chlorbenzol; Ether, wie Diethylether, Diisopropylether, Tetrahydrofuran, Dioxan oder 1,2-Dimethoxyethan. Ferner kann Essigsäure als Lösungsmittel verwendet werden.

Wird ohne Verdünnungsmittel gearbeitet, kann 2-Methyl-3-butanon im Überschuss eingesetzt werden. Normalerweise werden 1-10, vorzugsweise 1-4 Äquivalente Keton auf ein Äquivalent Aldehyd verwendet.

Die angewendete Temperatur liegt zwischen 0 und 25° C.

Der Fortgang der Reaktion kann durch $^1H$-NMR verfolgt werden. Die Reaktionszeiten liegen zwischen 4 und 24 h.

Die bei der Umsetzung der Aldehyde der Formel II mit dem Methylbutanon der Formel III erhältlichen 4,4-Dimethyl-3-halogen-1-hexen-5-one der Formel IV sind neu. Sie können isoliert und gereinigt oder ohne weitere Reinigung sofort in der nächsten Stufe weiter umgesetzt werden.

Bevorzugt sind Verbindungen der Formel IV, in der X und Y die für Verbindungen der Formel II angegebene bevorzugte und besonders bevorzugte Bedeutung haben.

Verbindungen der Formel V erhält man, indem man Verbindungen der Formel IV (oben) halogeniert.

Der Verlauf dieser Reaktion ist überraschend.

Es musste erwartet werden, dass neben einer Halogenierung der Methylgruppe bevorzugt an die Doppelbindung Halogen addiert wird. Auch eine Halogenierung in Allylstellung war nicht auszuschliessen, zumal durch das bereits vorhandene Halogenatom eine Aktivierung vorhanden ist.

Es ist somit als äusserst überraschend anzusehen, dass die Halogenierung äusserst selektiv unter den angegebenen Bedingungen praktisch ausschliesslich an der Methylgruppe erfolgt.

Verwendet man beispielsweise 4,4-Dimethyl-1,3-dichlor-1-(3,4-dichlorphenyl)-1-hexen-5-on als Ausgangsstoff und Brom als Halogenierungsmittel, so kann der Reaktionsablauf durch folgendes Formelschema wiedergegeben werden:

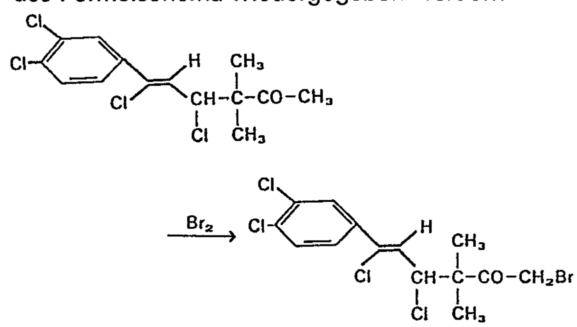


Die bei dem Verfahren verwendbaren Ausgangsstoffe sind durch die allgemeine Formel IV definiert. Darin haben Y und X die bevorzugte und besonders bevorzugte Bedeutung wie auf Seite 8 für die Verbindungen der Formel II angegeben.

Hal steht für Chlor oder Brom.

Als Halogenierungsmittel kommen Chlor, Brom oder Sulfurylchlorid in Frage.

Es wird üblicherweise in einem inerten Verdünnungsmittel gearbeitet. Als solche dienen beispielsweise Alkohole wie Methanol oder Ethanol, Chlorkohlenwasserstoffe, wie Methylenchlorid, Chloroform, Tetrachlorkohlenstoff, Dichlorethan oder hochchlorierte Aromaten, ferner Säuren, wie beispielsweise Ameisensäure oder Essigsäure oder Ester, wie Essigsäureester.

Ggf. kann in Gegenwart eines Katalysators, wie z. B. Halogenwasserstoffsäuren (Chlorwasserstoff, Bromwasserstoff) oder Lewis-Säuren (Aluminiumchlorid, Zinkchlorid, Aluminiumbromid) gearbeitet werden.

Die Reaktionstemperatur sollte +40° C nicht überschreiten, vorzugsweise wird zwischen −10 und +25° C gearbeitet.

Es wurde maximal bis zu einem Äquivalent Halogenierungsmittel zugegeben; es kann jedoch auch ein Unterschuss verwendet werden, um zu verhindern, dass ein zweites Halogenatom reagiert.

Die bei der Halogenierung der Verbindungen der Formel IV erhältlichen Verbindungen der Formel V sind neu. Sie können isoliert und gereinigt oder ohne weitere Reinigung sofort in der nächsten Stufe weiter umgesetzt werden. Bevorzugte und besonders bevorzugte Verbindungen der Formel V sind diejenigen, bei denen die Substituenten Y und X die bei den Verbindungen der Formel IV angegebenen bevorzugten Bedeutungen besitzen.

Verbindungen der Formel I (oben) erhält man, wenn man Verbindungen der Formel V mit Basen der Formel VI umsetzt.

Die Reaktion ist insofern als überraschend zu bezeichnen, da bekannt ist, dass ähnliche Verbindungen wie z. B.

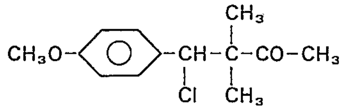

bereits unter äusserst milden Bedingungen mit wässerigen Basen oder Alkoholen und Carbonaten zu Alkoholen oder Ethern reagieren („Archiv der Pharmazie", *308*, 422 und *313*, 795):

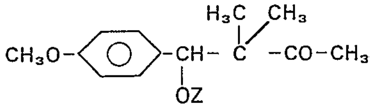

Z = H, Ethyl

Der Vorteil der erfindungsgemässen Reaktion liegt darin, dass sie von preiswerten, leicht zugänglichen Ausgangsprodukten ausgeht. Sie gestattet daher die Cyclopropancarbonsäureester der Formel I auf besonders wirtschaftliche Weise herzustellen.

Ein weiterer Vorteil liegt darin, dass mit dieser Reaktion eine stereoselektive Synthese von Verbindungen, die an der Vinyl-Doppelbindung unsymmetrisch substituiert sind, möglich ist.

Verwendet man beispielsweise 4,4-Dimethyl-1,3-dichlor-6-brom-1-(4-fluorphenyl)-1-hexen-5-on als Ausgangsstoff und Natriumhydroxid als Base, so kann der Reaktionsverlauf durch folgendes Formelschema wiedergegeben werden:

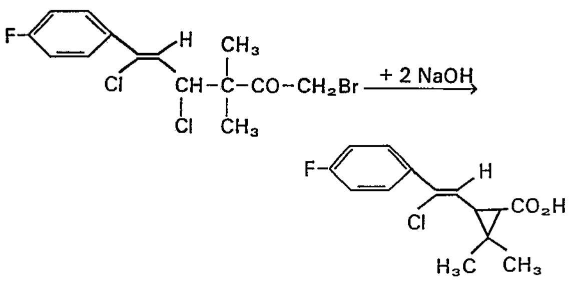


Die bei dem Verfahren verwendbaren Ausgangsstoffe sind durch die allgemeinen Formeln V und VI definiert. Darin haben Y, X und Hal die oben angegebene bevorzugte und besonders bevorzugte Bedeutung.

Im einzelnen seien als Beispiele für Basen genannt: Natriumhydroxid, Kaliumhydroxid, Calciumhydroxid, Natriummethylat, Natriumethylat, Natriumbutylat, Kalium-tert.-butylat.

Bevorzugt wird bei Verwendung der Hydroxide in Wasser und/oder einem inerten Verdünnungsmittel gearbeitet. Hier kommen beispielsweise Alkohole wie Methanol, Ethanol, t-Butanol, Ether wie Dioxan, Tetrahydrofuran, Dimethoxyethan oder Ketone wie Aceton sowie Dimethylformamid in Frage. Es können jedoch auch nicht mit Wasser mischbare Lösungsmittel, wie Methylenchlorid, Petrolether, Cyclohexan, Toluol oder Chlorbenzol, ggf. in Gegenwart eines Phasentransferkatalysators, verwendet werden.

Bei Verwendung der Alkoholate wird am günstigsten in den entsprechenden Alkoholen gearbeitet.

Es müssen mindestens 2 Äquivalente Base VI auf ein Mol Ausgangsstoff der Formel V eingesetzt werden. Ein Überschuss an Base bis zu 10 Äquivalenten ist meist vorteilhaft.

Die Reaktionstemperaturen können in einem grösseren Bereich variiert werden, jedoch gelingt die Reaktion überraschenderweise bereits unter äusserst schonenden Bedingungen.

Im allgemeinen arbeitet man zwischen 0 und 150° C, vorzugsweise jedoch zwischen 20 und 100° C.

Die Aufarbeitung des Reaktionsgemisches erfolgt bei Herstellung der Säuren (R=H) durch Extraktion im Alkalischen (zur Entfernung von Verunreinigungen) und nach Ansäuern der Wasserphase durch erneute Extraktion. Bei Herstellung von Estern erfolgt die Reinigung durch Destillation. Vorher wird mit Wasser verdünnt, neutral gestellt und extrahiert.

Unter den angegebenen Bedingungen entstehen cis/trans-Gemische der Säuren, in etwa im Verhältnis 25:75 bis 35:65. Es wurde jedoch überraschenderweise gefunden, dass unter bestimmten Bedingungen nahezu ausschliesslich die trans-Säuren erhalten werden. So gelingt es beispielsweise, sehr reine trans-3-[Z-2'-Chlor-2'-(p-Cl-phenyl)vinyl]-2,2-dimethyl-cyclopropancarbonsäure herzustellen, die nach dem bisherigen Stand der Technik nur durch Chlorierung und erneute Dechlorierung mit Metallen aus einem trans-E/Z-Säuregemisch herstellbar war (vgl. DE-OS Nr. 3035149).

Um zu möglichst reinen trans-Säuren zu gelangen, werden als Basen bevorzugt Kaliumhydroxid oder Natriumhydroxid verwendet; sie können auch Wasser enthalten; besonders bevorzugt ist die Verwendung von pulverisiertem technischem Kaliumhydroxid. Als Lösungsmittel werden Alkohole, wie Methanol, Ethanol, Propanol, Glykolmonomethylether, verwendet. Besonders bevorzugt ist Methanol.

Der cis-Anteil sinkt mit steigender Temperatur. Wählt man diese jedoch zu hoch, so sinkt die Ausbeute, beispielsweise durch Eliminierung von Chlorwasserstoff und Bildung einer Dreifachbindung. Es muss somit die für jede Säure optimale Temperatur gefunden werden. Diese liegt zwischen 20 und 150° C, bevorzugt jedoch zwischen 40 und 100° C.

*Beispiel 1:*

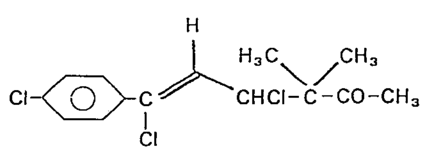

In eine Mischung aus 20,1 g (0,1 mol) 3-Chlor-3-(4-chlorphenyl)propenal und 34,4 g (0,4 mol) Methyl-isopropylketon wird bei 10° C bis zur Sättigung Chlorwasserstoff eingeleitet. Man lässt über Nacht stehen, giesst auf Wasser und stellt mit Soda neutral. Nach Extrahieren mit Methylenchlorid wird getrocknet und Methylenchlorid und überschüssiges Methylisopropylketon abdestilliert. Es hinterbleiben 24,9 g rohes 4,4-Dimethyl-1,3-dichlor-1-(4-chlorphenyl)-1-hexen-5-on, das durch Destillation im Hochvakuum gereinigt wird. Man erhält 22,2 g (72,7% der Theorie) eines farblosen oder schwach gelblichen Öls vom Kp = 150-155° C/0,1 mbar bzw. vom Fp = 42-43° C.

*Beispiel 2:*

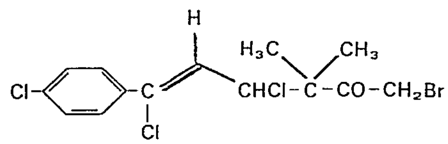

10 g (0,0325 mol) 4,4-Dimethyl-1,3-dichlor-1-(4-chlorphenyl)-1-hexen-5-on werden in 200 ml Chloroform gelöst und 5,2 g Brom (0,0325 mol) bei 20° C zugetropft. Man rührt 1 h bei Raumtemperatur nach und destilliert das Lösungsmittel ab. Man erhält 12,5 g eines Öls, das ganz überwiegend aus 4,4-Dimethyl-1,3-dichlor-6-brom-1-(4-chlorphenyl)-1-hexen-5-on besteht.

$^1H-NMR$ ($CDCl_3$) = $\delta$ = 1,5 ppm (6H, m); 4,2 ppm (2H, s); 5,23 ppm (1H, d, J = 5 Hz); 6,19 ppm (1H, d, J = 5 Hz); 7,2-7,6 ppm (4H, m).

*Beispiel 3:*

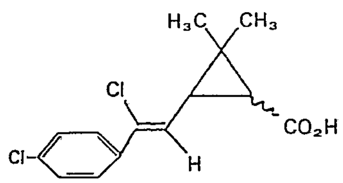

12 g (0,031 mol) 4,4-Dimethyl-1,3-dichlor-6-brom-1-(4-chlorphenyl)-1-hexen-5-on in 50 ml Dioxan werden zu einer Lösung von 12,45 g (0,31 mol) Natriumhydroxid in 115 ml Wasser und 100 ml Dioxan bei 20° C getropft. Man rührt 12 h bei Raumtemperatur nach, verdünnt mit 750 ml Wasser und extrahiert mit Methylenchlorid. Die Wasserphase wird mit Salzsäure angesäuert und ebenfalls mit Methylenchlorid extrahiert. Die sauren Extrakte werden getrocknet und das Methylenchlorid abdestilliert. Die letzten Lösungsmittelreste (Dioxan) werden durch Trocknen im Hochvakuum bei 60° C entfernt. Das zurückbleibende Produkt besteht aus cis/trans-3-(Z-2-Chlor-2-(4-chlor-phenyl)vinyl)-2,2-dimethyl-cyclopropancarbonsäure vom Schmelzpunkt 128-135° C.

*Beispiel 4:*

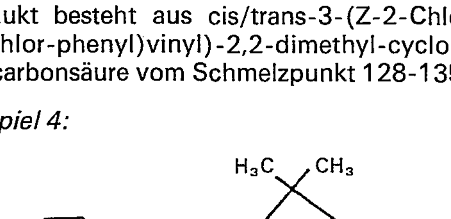

20 g (0,15 mol) Zimtaldehyd und 25,8 g (0,3 mol) Methylisopropylketon werden vorgelegt und bei 10° C trockner Chlorwasserstoff bis zur Sättigung eingeleitet. Man rührt 12 h bei 20-25° C nach, giesst auf Wasser und stellt mit Soda neutral. Nach Extraktion mit Methylenchlorid wird getrocknet und das Methylenchlorid abdestilliert. Man erhält 25,7 g (72,4% der Theorie) 1-Phenyl-3-chlor-4,4-dimethyl-1-hexen-5-on vom Schmelzpunkt 71° C.

*Beispiel 5:*

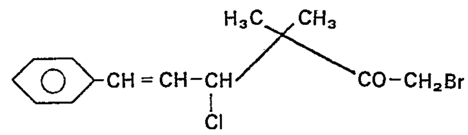

5 g (0,021 mol) 1-Phenyl-3-chlor-4,4-dimethyl-1-hexen-5-on werden in 40 ml Chloroform gelöst und 3,38 g (0,021 mol) Brom bei 20° C zugetropft. Nach 1 h wird das Lösungsmittel abdestilliert. Es bleiben 6,8 g eines Öls zurück, das hauptsächlich aus 1-Phenyl-3-chlor-6-brom-4,4-dimethyl-1-hexan-5-on besteht und direkt weiter umgesetzt wird.

*Beispiel 6:*

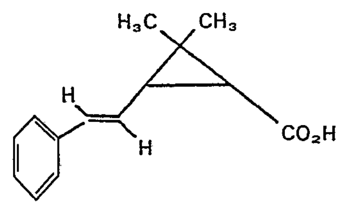

6 g rohes (aus Beispiel 5) 1-Phenyl-3-chlor-6-brom-4,4-dimethyl-1-hexen-5-on in 50 ml Dioxan wird zu einer Lösung aus 8,8 g Natriumhydroxid in 80 ml Wasser und 50 ml Dioxan getropft und 12 h bei 20° C gerührt. Dann verdünnt man mit Wasser und extrahiert mit Methylenchlorid. Die Wasserphase wird mit Salzsäure angesäuert und ebenfalls mit Methylenchlorid extrahiert. Die sauren Extrakte werden getrocknet und das Methylenchlorid abdestilliert. Die letzten Lösungsmittelreste (Dioxan) werden durch Trocknen im Hochvakuum bei 60° C entfernt. Das zurückbleibende Produkt besteht aus cis/trans-3-Styryl-2,2-dimethyl-cyclopropancarbonsäure. Die Struktur wird durch das $^1H-NMR$-Spektrum nachgewiesen.

*Beispiel 7:*

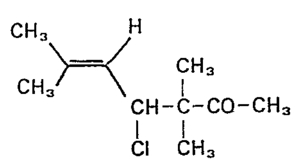

In eine Mischung aus 42 g (0,5 mol) 3,3-Dimethylacrolein und 86 g (1 mol) Methylisopropylketon wird bei 10° C bis zur Sättigung Chlorwasserstoff eingeleitet und anschliessend 12 h nachgerührt (ohne weitere Kühlung). Der Ansatz wird auf Eiswasser gegossen und mit Natriumcarbonat neutral gestellt. Man extrahiert dreimal mit Methylenchlorid, wäscht die vereinigten organischen Phasen mit Wasser und trocknet mit Natriumsulfat. Nach Abdestillieren des Methylenchlorids hinterbleiben 97 g eines Öls, das im Hochvakuum destilliert wird. Man erhält 59 g

(62,6% der Theorie) 2,5,5-Trimethyl-4-chlor-2-hepten-6-on vom Siedepunkt 70-80° C/0,05 mbar.

*Beispiel 8:*

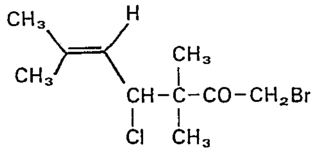

Man löst 6,03 g (0,032 mol) 2,5,5-Trimethyl-4-chlor-2-hepten-6-on in 75 ml Chloroform, versetzt mit 2 Tropfen etherischer Chlorwasserstofflösung und gibt dann 5,12 g (0,032 mol) Brom auf einmal bei 25° C hinzu. Man rührt 2,5 h bei Raumtemperatur nach und destilliert Bromwasserstoff und Lösungsmittel im Vakuum ab; die letzten Reste werden im Hochvakuum entfernt. Man erhält 8,7 g rohes 2,5,5-Trimethyl-4-chlor-7-brom-2-hepten-6-on, dessen Struktur durch das $^1H-NMR$-Spektrum gesichert wird. Es wird direkt in die nächste Stufe eingesetzt:

*Beispiel 8a:*

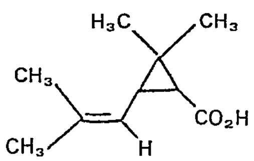

11,96 g (0,299 mol) Natriumhydroxid werden in 107,64 g Wasser gelöst und 8 g rohes 2,5,5-Trimethyl-4-chlor-7-brom-2-hepten-6-on (aus dem vorhergehenden Beispiel) in 25 ml Dioxan zugetropft und 12 h bei Raumtemperatur nachgerührt. Anschliessend wird mit Wasser verdünnt und dreimal mit Methylenchlorid extrahiert. Die Wasserphase wird angesäuert und ebenfalls dreimal mit Methylenchlorid extrahiert, getrocknet und eingeengt. Es hinterbleiben 3,2 g kristalliner Rückstand, der aus cis/trans-Chrysanthemumsäure besteht.

*Beispiel 9:*

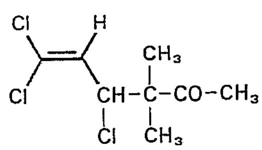

In eine Mischung aus 19 g (0,08 mol) Dichloracrolein und 27,52 g (0,32 mol) Methylisopropylketon wird bei 10° C bis zur Sättigung Chlorwasserstoff eingeleitet und anschliessend 12 h nachgerührt (ohne weitere Kühlung). Der Ansatz wird auf Eiswasser gegossen und mit Natriumcarbonat neutral gestellt. Man extrahiert dreimal mit Methylenchlorid, wäscht die vereinigten organischen Phasen mit Wasser und trocknet mit Natriumsulfat. Nach Abdestillieren des Lösungsmittels hinterbleiben 22 g 1,1,3-Trichlor-4,4-dimethyl-1-hexen-5-on, das im Hochvakuum destilliert wird. Siedepunkt: 80° C (0,05 mbar) $^1H-NMR$: $\delta$ ($CDCl_3$): 1,2 ppm (S, 6H), 2,2 ppm (S, 3H), 4,95 ppm (d, 1H), 6,05 ppm (d, 1H).

*Beispiel 10:*

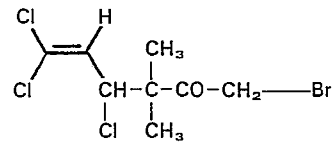

Man löst 11,475 g (0,05 mol) 1,1,3-Trichlor-4,4-dimethyl-1-hexen-5-on in 150 ml Chloroform und gibt 8 g (0,05 mol) Brom ohne Kühlung hinzu. Man rührt 3 h bei Raumtemperatur nach und destilliert Bromwasserstoff und Lösungsmittel im Vakuum ab; die letzten Reste werden im Hochvakuum entfernt. Man erhält 15,5 g rohes 1,1,3-Trichlor-6-brom-4,4-dimethyl-1-hexen-5-on, dessen Struktur durch das $^1H-NMR$-Spektrum gesichert wird. Es wird direkt in die nächste Stufe eingesetzt:

*Beispiel 10a:*

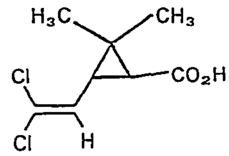

3,6 g (0,09 mol) Natriumhydroxid werden in 36,4 g Wasser gelöst und 9,255 g (0,03 mol) rohes 1,1,3-Trichlor-6-brom-4,4-dimethyl-1-hexen-5-on hinzugefügt. Dann erhitzt man 15 min auf 80° C und lässt abkühlen. Nach Verdünnen mit Wasser wird mit Methylenchlorid extrahiert. Die Wasserphase wird angesäuert und erneut extrahiert.

Nach Trocknen und Einengen erhält man 6,05 g cis/trans-Permethrinsäure. Ausbeute: 96,5%.

*Beispiel 11:*

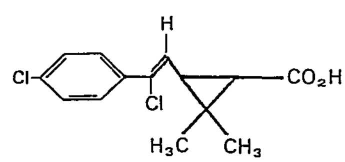
(Z-trans)

Man legt 52 ml einer 3N methanolischen KOH-Lösung (enthält 9,93 g = 0,156 mol 88%ige technische, pulverisierte KOH) vor und trägt bei 50° C 10 g rohes 4,4-Dimethyl-1,3-dichlor-6-brom-1-(4-chlorphenyl)-1-hexen-5-on ein. Nach 15 min lässt man abkühlen und rührt 6 h nach. Dann verdünnt man mit Wasser (ph-Wert: 11) und extrahiert dreimal mit Methylenchlorid. Die Wasserphase wird mit Salzsäure angesäuert und ebenfalls dreimal mit Methylenchlorid extrahiert. Die sauren Extrakte werden getrocknet und das Methylenchlorid abdestilliert. Die letzten Lösungsmittelreste werden durch Trocknen im Hochvakuum bei 60° C entfernt. Die zurückbleibende Säure (6,1 g) hat laut gaschromatographischer Analyse (silylierte Probe) einen Gehalt von 92,9%. Ausbeute: 90%. Durch einmaliges Umkristallisieren aus Cyclohexan wird reine trans-3-(Z-2-Chlor-2-arylvinyl)-2,2-dimethyl-cyclopropan-1-carbonsäure erhalten.

*Beispiel 12:*

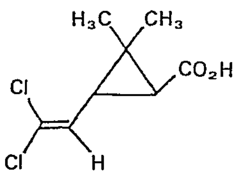

(trans)

Man legt 200 ml einer 3N methanolischen KOH-Lösung (enthält 38,2 g = 0,6 mol 88%ige technische, pulverisierte KOH) vor und trägt bei 60° C 30,85 g (0,1 mol) 1,1,3-Trichlor-6-brom-4,4-dimethyl-1-hexen-5-on ein. Nach 15 min lässt man abkühlen und rührt 10 h bei Raumtemperatur nach. Dann verdünnt man mit Wasser (pH-Wert: 11) und extrahiert dreimal mit Methylenchlorid. Die Wasserphase wird mit Salzsäure angesäuert und ebenfalls dreimal mit Methylenchlorid extrahiert. Die sauren Extrakte werden getrocknet und das Methylenchlorid abdestilliert. Die letzten Lösungsmittelreste werden durch Trocknen im Hochvakuum bei 60° C entfernt. Die zurückbleibende trans-2,2-Dichlorvinyl-3,3-dimethylcyclopropan-1-carbonsäure wiegt 19,65 g (94% Ausbeute der Theorie).

## Patentansprüche

1. Verfahren zur Herstellung von Verbindungen der Formel (I)

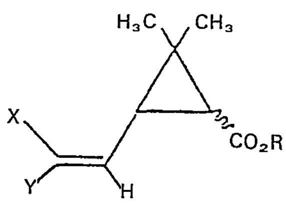

(I)

in welcher

Y für Halogen, Alkyl, Cycloalkyl, die ggf. durch Halogen oder $C_{1-4}$-Alkoxy substituiert sind, Alkenyl, das ggf. durch Halogen substituiert ist, für substituiertes oder unsubstituiertes Aryl oder Heteroaryl sowie für Alkoxycarbonyl steht,

X für Wasserstoff, Halogen oder ggf. durch Halogen substituiertes Alkyl steht,

wobei X und Y gemeinsam mit dem angrenzenden C-Atom einen gesättigten cycloaliphatischen Ring mit bis zu 6 C-Atomen bilden können, und

R für Wasserstoff oder $C_1$-$C_4$-Alkyl steht, dadurch gekennzeichnet, dass man Aldehyde der Formel (II)

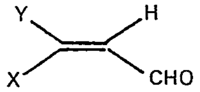

(II)

in welcher

Y und X die oben angegebene Bedeutung besitzen,

mit 2-Methyl-butan-3-on (III)

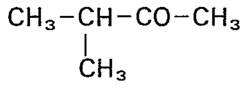

(III)

in Anwesenheit von Halogenwasserstoffsäuren umsetzt, die dabei erhaltenen 4,4-Dimethyl-3-halogen-1-hexen-5-one der Formel (IV)

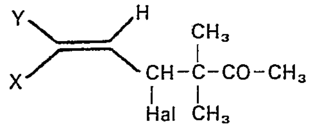

(IV)

in welcher

Y und X die oben angegebene Bedeutung haben, und

Hal für Chlor oder Brom steht,

halogeniert und die dabei erhaltenen Verbindungen der Formel (V)

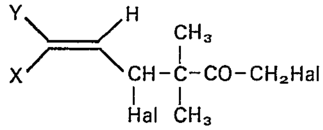

(V)

in welcher

Y, X und Hal die oben angegebene Bedeutung besitzen, wobei die beiden Hal unabhängig voneinander sind,

mit Basen der Formel (VI)

R–OM (VI)

in welcher

R die oben angegebene Bedeutung besitzt und

M für ein Äquivalent eines Alkali- oder Erdalkalimetallions steht,

umsetzt.

2. 4,4-Dimethyl-3-halogen-1-hexen-5-one der Formel (IV)

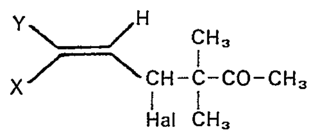

(IV)

in welcher

Y für Halogen, Alkyl, Cycloalkyl, die ggf. durch Halogen oder $C_{1-4}$-Alkoxy substituiert sind, Alkenyl, das ggf. durch Halogen substituiert ist, für substituiertes oder unsubstituiertes Aryl oder Heteroaryl sowie für Alkoxycarbonyl steht,

X für Wasserstoff, Halogen oder ggf. durch Halogen substituiertes Alkyl steht,

wobei X und Y gemeinsam mit dem angrenzenden C-Atom für einen gesättigten Cycloaliphatischen Ring mit bis zu 6 C-Atomen stehen können, und

Hal für Chlor oder Brom steht.

3. Verfahren zur Herstellung von 4,4-Dimethyl-3-halogen-1-hexen-5-onen der Formel (IV)

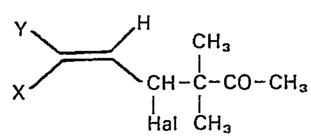

(IV)

in welcher

Y, X und Hal die in Anspruch 2 angegebene Bedeutung haben, dadurch gekennzeichnet, dass man Aldehyde der Formel (II)

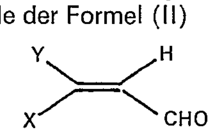

(II)

in welcher

Y und X die oben angegebene Bedeutung haben,

mit 2-Methyl-butan-3-on der Formel (III)

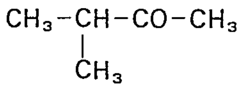
(III)

in Anwesenheit von Halogenwasserstoffen umsetzt.

4. Verbindungen der Formel (V)

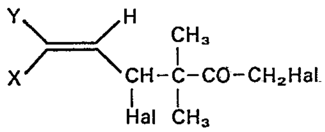
(V)

in welcher

Y für Halogen, Alkyl, Cycloalkyl, die ggf. durch Halogen oder $C_{1-4}$-Alkoxy substituiert sind, Alkenyl, das ggf. durch Halogen substituiert ist, für substituiertes oder unsubstituiertes Aryl oder Heteroaryl sowie für Alkoxycarbonyl steht,

X für Wasserstoff, Halogen oder ggf. durch Halogen substituiertes Alkyl steht,

wobei X und Y gemeinsam mit dem angrenzenden C-Atom für einen gesättigten cycloaliphatischen Ring mit bis zu 6 C-Atomen stehen können, und die beiden

Hal unabhängig voneinander für Chlor oder Brom stehen.

5. Verfahren zur Herstellung der Verbindungen der Formel (V)

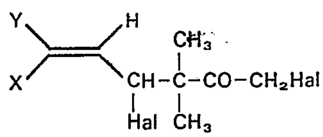
(V)

in welcher

Y, X und Hal die in Anspruch 4 angegebene Bedeutung haben, dadurch gekennzeichnet, dass man 4,4-Dimethyl-3-halogen-1-hexen-5-one der Formel (IV)

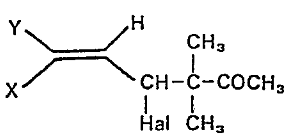
(IV)

in welcher

Y, X und Hal die oben angegebene Bedeutung haben, halogeniert.

6. Verfahren zur Herstellung von Verbindungen der Formel (I)

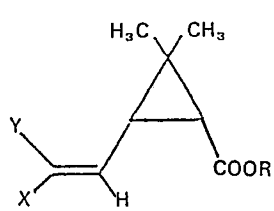
(I)

in welcher

Y, X und R die in Anspruch 1 angegebene Bedeutung haben, dadurch gekennzeichnet, dass man Verbindungen der Formel (V)

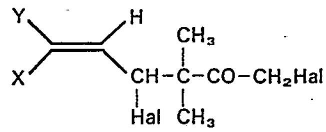
(V)

in welcher

Y, X und Hal die in Anspruch 1 angegebene Bedeutung haben,

mit Basen der Formel (VI)

R−OM (VI)

in welcher

R und M die in Anspruch 1 angegebene Bedeutung haben, umsetzt.

7. Verfahren gemäss Anspruch 7, dadurch gekennzeichnet, dass man Verbindungen der Formel (V) mit Natrium- oder Kaliumhydroxid in Gegenwart von Alkoholen zwischen 20 und 150° C umsetzt.

8. Verfahren gemäss Anspruch 7, dadurch gekennzeichnet, dass man Verbindungen der Formel (V) mit Natrium- oder Kaliumhydroxid in Gegenwart von Methanol zwischen 40 und 100° C

## Claims

1. Process for the preparation of compounds of the Formula (I)

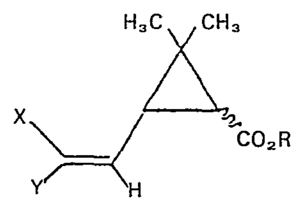
(I)

in which

Y represents halogen, alkyl or cycloalkyl which are optionally substituted by halogen or $C_{1-4}$-alkoxy, alkenyl which is optionally substituted by halogen, substituted or unsubstituted aryl or heteroaryl and alkoxycarbonyl,

X represents hydrogen, halogen or optionally halogen-substituted alkyl,

it being possible for X and Y, together with the adjacent C atom, to form a saturated cycloaliphatic ring with up to 6 C atoms, and

R represents hydrogen or $C_1$-$C_4$-alkyl, characterised in that aldehydes of the Formula (II)

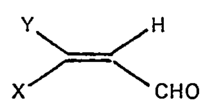
(II)

in which

Y and X have the meaning indicated above, are reacted with 2-methyl-butan-3-one (III)

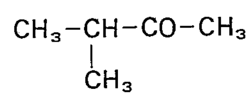
(III)

in the presence of hydrohalic acids, the 4,4-dimethyl-3-halogeno-1-hexen-5-ones thereby obtained, of the Formula (IV)

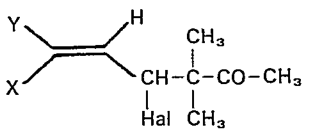

(IV)

in which

Y and X have the meaning indicated above and
Hal represents chlorine or bromine,

are halogenated and the compounds thereby obtained, of the Formula (V)

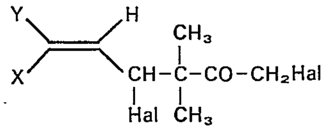

(V)

in which

Y, X and Hal have the meaning indicated above, the two Hal's being independent of each other, are reacted with bases of the Formula (VI)

R−OM (VI)

in which

R has the meaning indicated above and
M represents one equivalent of an alkali or alkaline earth metal ion.

2. 4,4-Dimethyl-3-halogeno-1-hexen-5-ones of the Formula (IV)

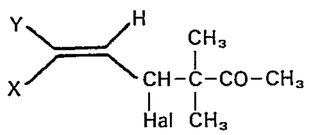

(IV)

in which

Y represents halogen, alkyl or cycloalkyl which are optionally substituted by halogen or $C_{1-4}$-alkoxy, alkenyl which is optionally substituted by halogen, substituted or unsubstituted aryl or heteroaryl and alkoxycarbonyl,

X represents hydrogen, halogen or optionally halogen-substituted alkyl,

it being possible for X and Y, together with the adjacent C atom, to represent a saturated cycloaliphatic ring with up to 6 C atoms, and

Hal represents chlorine or bromine.

3. Process for the preparation of 4,4-dimethyl-3-halogeno-1-hexen-5-ones of the Formula (IV)

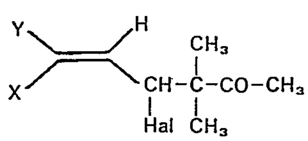

(IV)

in which

Y, X and Hal have the meaning indicated in Claim 2, characterised in that aldehydes of the Formula (II)

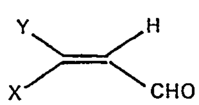

(II)

in which

Y and X have the meaning indicated above,

are reacted with 2-methyl-butan-3-one of the Formula (III)

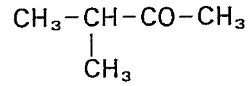

(III)

in the presence of hydrogen halides.

4. Compounds of the Formula (V)

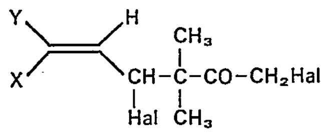

(V)

in which

Y represents halogen, alkyl or cycloalkyl which are optionally substituted by halogen or $C_{1-4}$-alkoxy, alkenyl which is optionally substituted by halogen, substituted or unsubstituted aryl or heteroaryl and alkoxycarbonyl,

X represents hydrogen, halogen or optionally halogen-substituted alkyl,

it being possible for X and Y, together with the adjacent C atom, to represent a saturated cycloaliphatic ring with up to 6 C atoms,

and the two Hal's, independently of each other, represent chlorine or bromine.

5. Process for the preparation of the compounds of the Formula (V)

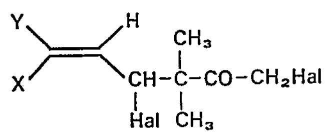

(V)

in which

Y, X and Hal have the meaning indicated in Claim 4, characterised in that 4,4-dimethyl-3-halogeno-1-hexen-5-ones of the Formula (IV)

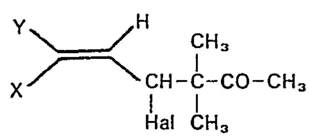

(IV)

in which

Y, X and Hal have the meaning indicated above, are halogenated.

6. Process for the preparation of compounds of the Formula (I)

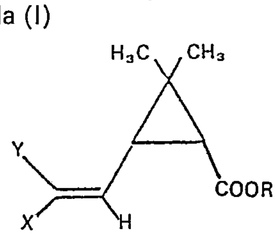

(I)

in which

Y, X and R have the meaning indicated in Claim 1, characterised in that compounds of the Formula (V)

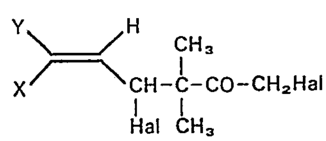

(V)

in which

Y, X and Hal have the meaning indicated in Claim 1, are reacted with bases of the Formula (VI)

R−OM (VI)

in which

R and M have the meaning indicated in Claim 1.

7. Process according to Claim 7, characterised in that compounds of the Formula (V) are reacted with sodium hydroxide or potassium hydroxide in the presence of alcohols at between 20 and 150° C.

8. Process according to Claim 7, characterised in that compounds of the Formula (V) are reacted with sodium hydroxide or potassium hydroxide in the presence of methanol at between 40 and 100° C.

## Revendications

1. Procédé de production de composés de formule (I)

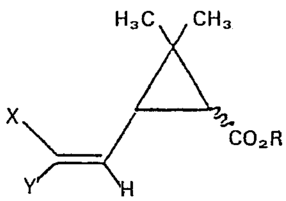

(I)

dans laquelle

Y représente un halogène, un groupe alkyle, un groupe cycloalkyle, qui sont éventuellement substitués par un halogène ou un radical alkoxy en $C_1$ à $C_4$, un groupe alcényle qui est éventuellement substitué par un halogène, un groupe aryle ou hétéroaryle substitué ou non substitué ainsi qu'un groupe alkoxycarbonyle,

X représente l'hydrogène, un halogène ou un groupe alkyle éventuellement substitué par un halogène,

X et Y pouvant former conjointement avec l'atome adjacent de carbone un noyau cycloaliphatique saturé ayant jusqu'à 6 atomes de carbone, et

R représente l'hydrogène ou un groupe alkyle en $C_1$ à $C_4$, caractérisé en ce qu'on fait réagir des aldéhydes de formule (II)

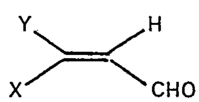

(II)

dans laquelle

Y et X possèdent la définition indiquée ci-dessus, avec la 2-méthyl-butane-3-one (III)

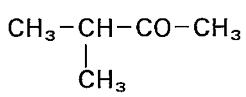

(III)

en présence d'acides halogénhydriques, on halogène les 4,4-diméthyl-3-halogéno-1-hexène-5-ones ainsi obtenues de formule (IV)

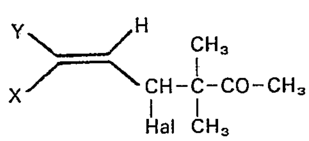

(IV)

dans laquelle

Y et X ont la définition indiquée ci-dessus, et

Hal représente le chlore ou le brome, et on fait réagir les composés ainsi obtenus de formule (V)

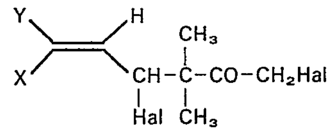

(V)

dans laquelle

Y, X et Hal possèdent la définition indiquée ci-dessus, les deux Hal étant indépendants l'un de l'autre,

avec des bases de formule (VI)

R−OM (VI)

dans laquelle

R possède la définition indiquée ci-dessus, et

M est un équivalent d'un ion de métal alcalin ou de métal alcalino-terreux.

2. 4,4-Diméthyl-3-halogéno-1-hexène-5-ones de formule (IV)

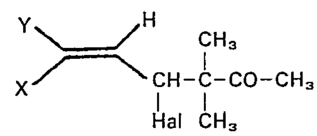

(IV)

dans laquelle

Y est un halogène, un groupe alkyle, un groupe cycloalkyle, qui sont éventuellement substitués par un halogène ou un radical alkoxy en $C_1$ à $C_4$, un groupe alcényle qui est éventuellement substitué par un halogène, un groupe aryle ou hétéroaryle substitué ou non substitué ainsi qu'un groupe alkoxycarbonyle,

X représente l'hydrogène, un halogène ou un groupe alkyle éventuellement substitué par un halogène,

X et Y pouvant former conjointement avec l'atome adjacent d'azote un noyau cycloaliphatique saturé ayant jusqu'à 6 atomes de carbone, et

Hal désigne le chlore ou le brome.

3. Procédé de production de 4,4-diméthyl-3-halogéno-1-hexène-5-ones de formule (IV)

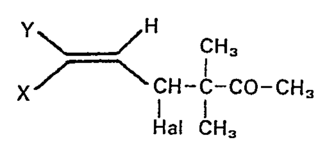

(IV)

dans laquelle

Y, X et Hal ont la définition indiquée dans la revendication 2, caractérisé en ce qu'on fait réagir des aldéhydes de formule (II)

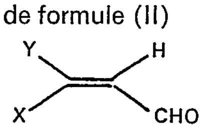

(II)

dans laquelle

Y et X ont la définition indiquée ci-dessus,

avec la 2-méthyl-butane-3-one de formule (III)

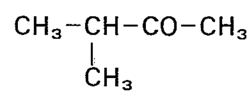

(III)

en présence d'halogénures d'hydrogène.

4. Composés de formule (V)

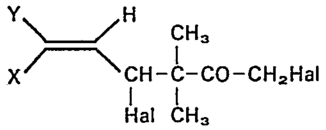

(V)

dans laquelle

Y désigne un halogène, un groupe alkyle, un groupe cycloalkyle, qui sont éventuellement substitués par un halogène ou par un radical alkoxy en $C_1$ à $C_4$, un groupe alcényle qui est éventuellement substitué par un halogène, un groupe aryle ou hétéroaryle substitué ou non substitué ainsi qu'un groupe alkoxycarbonyle,

X est l'hydrogène, un halogène ou un groupe alkyle éventuellement substitué par un halogène,

X et Y pouvant former conjointement avec l'atome adjacent de carbone un noyau cycloaliphatique saturé ayant jusqu'à 6 atomes de carbone, et les deux

Hal représentant indépendamment l'un de l'autre du chlore ou du brome.

5. Procédé de production des composés de formule (V)

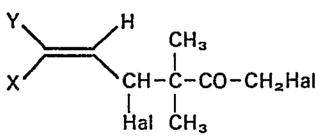

(V)

dans laquelle

Y, X et Hal ont la définition indiquée dans la revendication 4, caractérisé en ce qu'on halogène les 4,4-diméthyl-3-halogéno-1-hexène-5-ones de formule (IV)

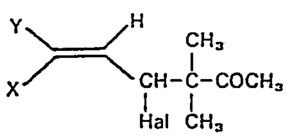

(IV)

dans laquelle

Y, X et Hal ont la définition indiquée ci-dessus.

6. Procédé de production de composés de formule (I)

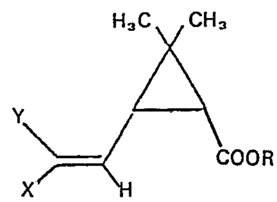

(I)

dans laquelle

Y, X et R ont la définition indiquée dans la revendication 1, caractérisé en ce qu'on fait réagir des composés de formule (V)

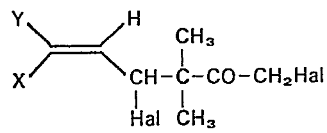

(V)

dans laquelle

Y, X et Hal ont la définition indiquée dans la revendication 1,

avec des bases de formule (VI)

R−OM (VI)

dans laquelle

R et M ont la définition indiquée dans la revendication 1.

7. Procédé suivant la revendication 7, caractérisé en ce qu'on fait réagir des composés de formule (V) avec l'hydroxyde de sodium ou de potassium en présence d'alcools, entre 20 et 150° C.

8. Procédé suivant la revendication 7, caractérisé en ce qu'on fait réagir des composés de formule (V) avec l'hydroxyde de sodium ou de potassium en présence de méthanol, entre 40 et 100° C.